# EUROPEAN PATENT SPECIFICATION

(11) **EP 2 626 355 B1**
(45) Date of publication and mention of the grant of the patent: **03.02.2016**
(21) Application number: 12275010.2
(22) Date of filing: 09.02.2012
(51) Int. Cl.: C07D 401/14, A61K 31/4178

(54) **Process for the preparation of nilotinib hydrochloride**
Verfahren zur Herstellung von Nilotinibhydrochlorid
Procédé de préparation d'hydrochlorure de nilotinib

(43) Date of publication of application: 14.08.2013
(73) Proprietor: Natco Pharma Limited, Hyderabad 500 033, Andhra Pradesh (IN)
(72) Inventor: Kompella, Amala, 500 033 Andhra Pradesh (IN); Bhujanga Rao, Adibhatla Kali Satya, 500 033 Andhra Pradesh (IN); Rachakonda, Sreenivas, 500 033 Andhra Pradesh (IN); Gampa, Venugopala Krishna, 500 033 Andhra Pradesh (IN); Nannapaneni, Venkaiah Chowdary, 500 033 Andhra Pradesh (IN)
(74) Representative: Srinivasan, Ravi Chandran

(56) References cited:
- WO-A1-2010/060074
- WO-A1-2011/163222
- WO-A2-2007/015870
- WO-A2-2010/054056
- WEI-SHENG HUANG ET AL: "An Efficient Synthesis of Nilotinib (AMN107)", SYNTHESIS, GEORG THIEME VERLAG, STUTTGART, DE, no. 14, 1 January 2007 (2007-01-01), pages 2121-2124, XP002572746, ISSN: 0039-7881, DOI: 10.1055/S-2007-983754 [retrieved on 2007-07-03]

## Description

### Field of the invention

The present invention is concerned with a process for the preparation of Nilotinib hydrochloride dihydrate.

### Background of the invention

Nilotinib is the compound 4-methyl-N[3-(4-methyl-1H-imidazol-1-yl-5-(trifluoromethyl)phenyl]-3-{{4-(3-pyridinyl)-2-pyrimidinyl]amino]benzamide and is of Formula (I): 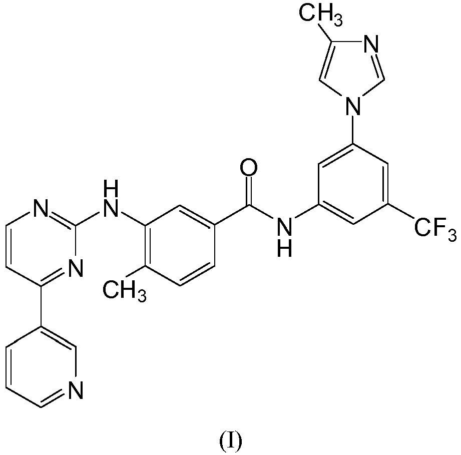 Nilotinib is a protein tyrosine kinase inhibitor.

The preparation of Nilotinib and the use thereof, especially as an antitumor agent, is described in WO 2004/005281 (Novartis Pharma) which was published on 15 January 2004.

WO 2010/060074 describes the preparation of Nilotinib and intermediates thereof. Indian Patent Application No. 768/CHE/2010 describes synthesis of Nilotinib free base.

### Summary of the invention

The present inventors have devised a new process for preparing Nilotinib hydrochloride. Nilotinib hydrochloride prepared via the process of the invention exists in a stable crystal form. This form is retained, even under accelerated stress conditions (for example an elevated temperature of 40°C combined with relative humidity of 75%). It is particularly surprising that the present process allows preparation of a stable form of Nilotinib hydrochloride dihydrate, which was previously believed to be unstable.

Accordingly, the present invention relates to a process for the preparation of a hydrochloride dihydrate salt of a compound of formula (I): 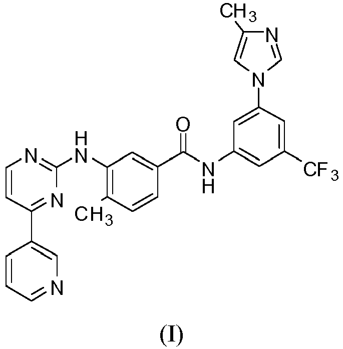 which process comprises converting a compound of formula (IV): 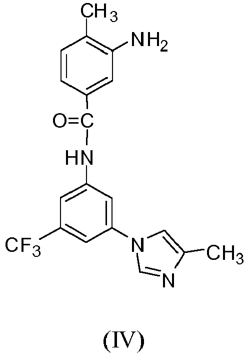 or a pharmaceutically acceptable salt thereof, into the hydrochloride dihydrate salt of the compound of formula (I), wherein hydrochloric acid in methanol and water medium is used to form the hydrochloride dihydrate salt.

### Brief description of the figures

Figure 1 shows the X-ray powder diffraction pattern for the Nilotinib hydrochloride prepared in Example 1(e).
Figure 2 shows the X-ray powder diffraction pattern for the Nilotinib hydrochloride formulation prepared in Example 3.

### Detailed description of the invention

The term "pharmaceutically acceptable salt" typically refers to a salt prepared from an acid which is acceptable for administration to a patient, such as a mammal. Such salts can be derived from from pharmaceutically acceptable inorganic or organic acids.

Salts derived from pharmaceutically acceptable acids include acetic, benzenesulfonic, benzoic, camphosulfonic, citric, ethanesulfonic, formic, fumaric, gluconic, glutamic, hydrobromic, hydrochloric, hydrofluoric, lactic, maleic, malic, mandelic, methanesulfonic, trifluoroacetic, mucic, nitric, pantothenic, phosphoric, succinic, sulfuric, tartaric, p toluenesulfonic, xinafoic (1 hydroxy 2 naphthoic acid), napadisilic (1,5 naphthalenedisulfonic acid), triphenyl acetic and the like. Particularly preferred are salts derived from formic, fumaric, hydrobromic, hydrochloric, hydrofluoric, acetic, sulfuric, methanesulfonic, trifluoroacetic, xinafoic, tartaric, maleic, succinic and napadisilic acids. A particularly preferred pharmaceutically acceptable salt is hydrochloride.

An illustrative example of an overall process of the present invention is depicted in Scheme 2 below. As a skilled person will appreciate, each of intermediate compounds (II), (IIA), (XI), (III), (IV), (V) and (VI) may be used in free base form or in the form of a pharmaceutically acceptable salt. Generally the free base form is preferred. However, as depicted in the illustrative process below, it may be preferable, for example, to use the intermediate compound of formula (III) in the form of salt such as the hydrochloride salt.

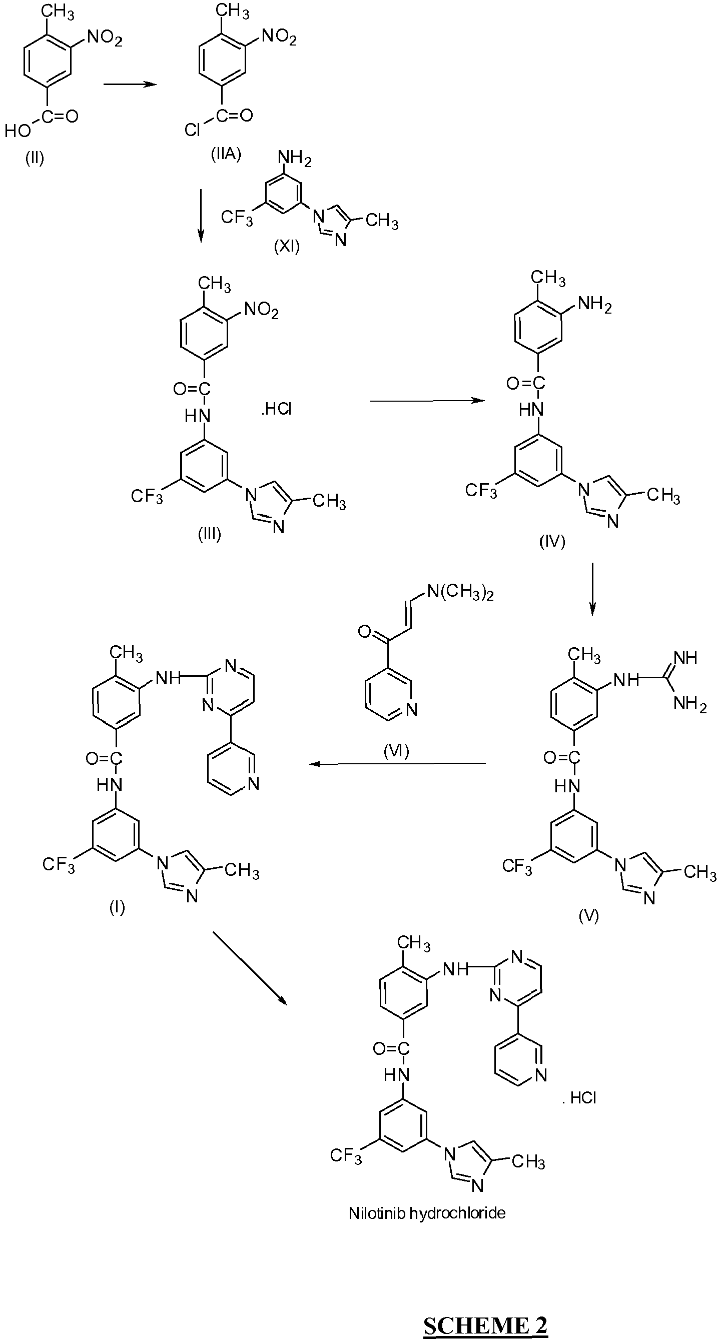

Each step of the process of the present invention is discussed in further detail below.

The compound of formula (II) is 4-methyl-3-nitro benzoic acid. Preferably, the compound of formula (II) is chlorinated, to give the compound of formula (IIA), which is 4-methyl-3-nitro benzoyl chloride:

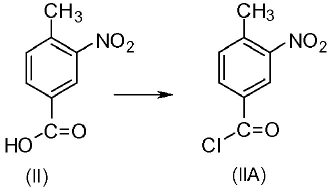

Chlorination can be achieved by conventional methods known to those skilled in the art. Preferably, chlorination is performed using SOCl₂ as the chlorinating agent.

The compound of formula (IIA) is then preferably reacted with a compound of formula (XI), to provide the compound of formula (III), which is 4-methyl-N-[3-(4-methyl-1H-imidazol-1-yl-5-(trifluoromethyl)phenyl]-3-nitro-benzamide:

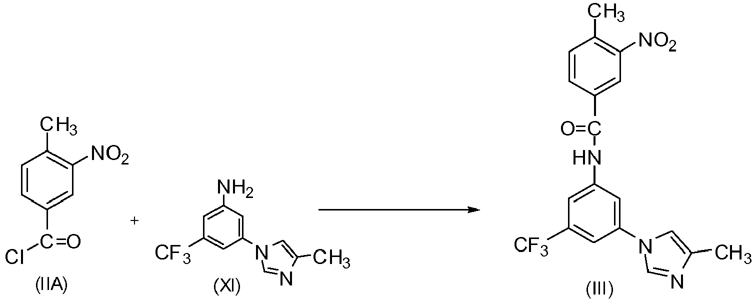

The compound of formula (XI) is 5-(4-methyl-1H-imidazol-1-yl)-3-(trifluoromethyl)-benzeneamine and can be obtained by conventional methods known to those skilled in the art.

The reaction of the compound of formula (IIA) with the compound of formula (XI) is typically carried out in the present of an alkali, preferably selected from sodium hydroxide or potassium hydroxide more preferably potassium hydroxide. The reaction of the compound of formula (IIA) with the compound of formula (XI) is typically carried out in a chlorinated hydrocarbon solvent, preferably methylene chloride or chloroform, more preferably chloroform. The reaction of the compound of formula (IIA) with the compound of formula (XI) is typically carried out at a temperature of 20-50°C, preferably 30-40°C.

The compound of formula (III) may be obtained, and then processed in subsequent steps of the process, in free base form or in salt form, such as hydrochloride salt form.

The compound of formula (III) is then reduced, to give a compound of formula (IV), which is 4-methyl-N-[3-(4-methyl-1H-imidazol-1-yl-5-(trifluoromethyl)phenyl]-3-aminobenzamide.

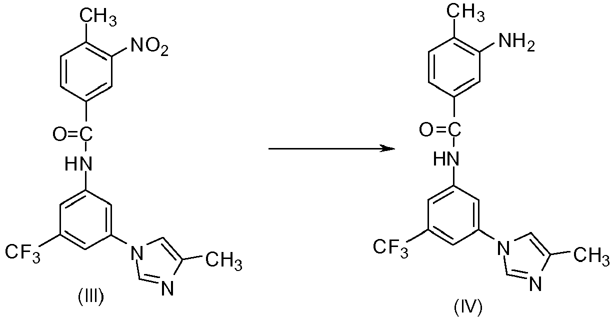

The reduction of the compound of formula (III) can be conducted using using standard reduction conditions and reagents, which will be known to one skilled in the art.

For example, the reduction of the compound of formula (III) can be achieved using stannous chloride (SnCl₂). Typically, stannous chloride in a methanol solvent is used. A preferred reaction time is 2 to 3 hours.

Alternatively, the reduction of the compound of formula (III) can be achieved using by catalytic hydrogenation, typically using Raney nickel, preferably in a methanol medium.

The compound of formula (IV) is then converted into a compound of fomula (I).

Typically this conversion is achieved by in a two-step transformation. Firstly, the compound of formula (IV) is reacted with cyanamide to obtain a compound of formula (V), which is 4-methyl-N-[3-(4-methyl-1H-imidazol-1-yl-5-(trifluoromethyl)phenyl]-3-guanidino-benzamide.

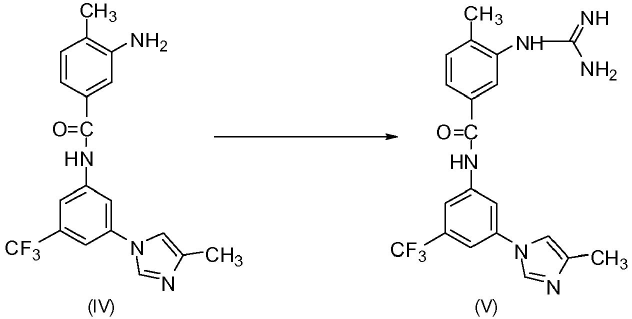

Typically, the cyanamide is dissolved in a C₁-C₆ alcohol, preferably n-butanol. Typically, the reaction temperature is 90-95°C, particularly when the solvent is n-butanol.

The compound of formula (V) is typically then reacted with a compound of formula (VI), to provide the compound of formula (I).

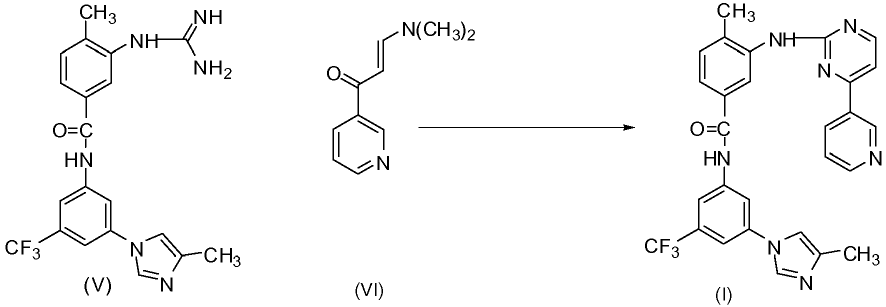

The compound of formula (VI) is 3-dimethylamino-1-(3-pyridyl)-2-propen-1-one. Typically, this reaction occurs in the presence of a base. Reflux conditions and temperature are preferably used.

The compound of formula (I) is then converted into a hydrochloride salt form of formula (I*): 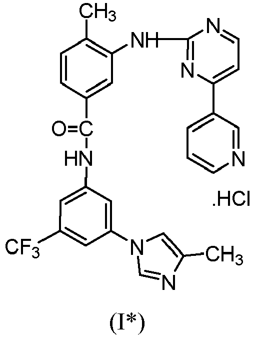 The compound of formula (I) is converted into the hydrochloride salt by reaction with hydrochloric acid. Hydrochloric acid, typically concentrated hydrochloric acid, in methanol and water medium is used to form the hydrochloride salt.

The hydrochloride salt of the compound of formula (I) is in the form of the dihydrate.

The moisture content of the compound obtainable by the process of the invention is typically less than 10% by weight, preferably less than 7% by weight, more preferably less than 6% by weight, for example about 5% by weight.

The hydrochloride salt of the compound of formula (I) prepared by the process of the present invention exists in a stable crystalline form. The crystalline form of the hydrochloride salt of the compound of formula (I) obtainable by the process of the invention has an X-ray powder diffraction pattern with one or more peaks at 4.3, 8.7, 9.5, 11.3, 13.2, 14.4, 17.3, 18.6, 19.3, 20.8, 22.2 and 25.3 degrees 2θ (± 0.1 degrees 2θ). Preferably two or more such peaks are observed, more preferably three or more, more preferably four or more, more preferably five or more. Most preferably all such peaks are observed.

It is particularly preferred that the crystalline form has an X-ray powder diffraction pattern with one or more peaks at 8.7, 17.3, 19.3, 22.2 and 25.3 degrees 20 (± 0.1 degrees 2θ). Preferably two or more such peaks are observed, more preferably three or more, more preferably four or more. Most preferably all such peaks are observed.

Typically, the peak at 8.7 degrees 2θ (± 0.1 degrees 2θ) is most intense. Preferably, the peak at 8.7 degrees 2θ (± 0.1 degrees 2θ) is at least twice as intense as the next most intense peak.

Preferably the crystalline form is in the form of the dihydrate

The crystalline form is thermally stable.

Typically, the X-ray powder diffraction pattern of a sample of the crystalline form which has been stored at 70°C for 40 hours is identical, or substantially identical, to that of the crystalline form of the invention described above. Preferably, at least 90% by weight, more preferably 95%, more preferably 98%, of a sample of the crystalline form of the invention which has been stored at 70°C for 40 hours retains the crystalline form of the invention described above.

Typically, the X-ray powder diffraction pattern of a sample of the crystalline form which has been stored at 40 °C and 75% relative humidity for 3 months is identical, or substantially identical, to that of the crystalline form of the invention described above. Preferably, at least 90% by weight, more preferably 95%, more preferably 98%, of a sample of the crystalline form of the invention which has been stored at 40 °C and 75% relative humidity for 3 months retains the crystalline form of the invention described above.

Typically, the crystalline form is not hygroscopic. Typically, the moisture content of the crystalline form after storage for 60 days at 40 °C and 75% relative humidity is less than 10% by weight, more preferably less than 7% by weight, and most preferably less than 6.5% by weight.

The pharmaceutical composition comprises a compound of the invention in association with a pharmaceutically acceptable diluent or carrier. The purpose of a pharmaceutical composition is to facilitate administration of a compound to an organism. As used herein, a physiologically/pharmaceutically acceptable diluent or carrier refers to a carrier or diluent that does not cause significant irritation to an organism and does not abrogate the biological activity and properties of the administered compound. Suitable pharmaceutically acceptable carriers and diluents are well known to those skilled in the art.

The details of the invention are provided in the Examples given below which are provided to illustrate the invention only and therefore they should not be construed to limit the scope of the invention

### Example 1

### (a) Preparation of 4-methyl-N-[3-(4-methyl-1H-imidazol-1-yl-5-(trifluoromethyl)phenyl]-3-nitro-benzamide hydrochloride of the formula (III)

To a suspension of 100g (0.55moles) of 3-amino-4-methylbenzoic acid of the formula (II) in chloroform (1L) of thionyl chloride 131.5g (1.10moles) and dimethyl formamide (1ml) were added. The reaction mass was heated to reflux temperature and maintained at the same temperature for 3 hours. Solvent chloroform was distilled off completely under vacuum and again 500ml of chloroform was charged and distilled under vacuum to remove traces of thionyl chloride. The residual 3-amino-4-methylbenzoyl chloride of the formula (IIA) was taken to the following condensation step.

5-(4-methyl-1H-imidazol-1-yl)-3-(trifluoromethyl)-benzeneamine 110g (0.45moles) of formula (XI) was dissolved in 1L of chloroform. 4-methyl-3-nitro benzoyl chloride of the formula (IIA) prepared above was added slowly to the reaction mass at 10-15°C for 60 minutes. Reaction mass was raised to room temperature and maintained for 4 hours. Reaction mass was filtered, washed with chloroform and dried.
Yield: 161 g (87%)
Purity: 99% (by HPLC)
IR and NMR were consistent with the proposed structure.

### (b) Preparation of 4-methyl-N-[3-(4-methyl-1H-imidazol-1-yl-5-(trifluoromethyl)phenyl]-3-amino-benzamide of the formula (IV)

To a chilled solution (10-15°C) of stannous chloride 265.6g (1.18moles) in 400ml methanol of 4-methyl-N-[3-(4-methyl-1H-imidazol-1-yl-5-(trifluoromethyl)phenyl]-3-nitro-benzamide hydrochloride 130g (0.29moles) of the formula (III) obtained from step (a) was added during 30 minutes. The reaction mass was maintained at 10-15°C for one hour and heated to reflux temperature. The reaction mass was maintained at reflux temperature for 90 minutes and brought to room temperature. Purified water (600ml) was charged to reaction mass at room temperature and maintained under stirring for 6-8 hours. Reaction mass was filtered, washed with 1N HCl and suck dried for 2 hours. Wet solid was charged into 2L purified water, cooled to 10-15°C and basified with 4% aqueous sodium hydroxide solution. Reaction mass was brought to room temperature and maintained at the same temperature for 2 hours, filtered and dried at 50-60°C to yield compound of formula (IV).
Yield: 104g (80%)
Purity: 99.4 % (by HPLC)

Alternatively compound of formula (IV) can be prepared by the catalytic hydrogenation of compound of formula (III) with Raney nickel in methanol medium as follows :
Into hydrogenation kettle 50g (0.123moles) of 4-methyl-N-[3-(4-methyl-1H-imidazol-1-yl-5-(trifluoromethyl) phenyl]-3-nitro-benzamide hydrochloride of the formula (III) obtained from step (a) was charged into 250ml methanol. Raney nickel (20g) was charged into pressure reactor kettle and hydrogenated at a hydrogen gas pressure of 60psi for 22 hours. After reaction completion reaction mass was filtered and washed with methanol. To the filtrate aqueous ammonia solution (45ml) was charged and stirred for 30 minutes. Reaction mass was filtered, washed thoroughly with purified water and dried at 50-60°C to yield compound of formula (IV)
   Yield: 35g(70%)
   Purity: 99.0 %(by HPLC)
   IR: 210-212°C
   IR and NMR were consistent with the proposed structure.

### (c) Preparation of 4-methyl-N-[3-(4-methyl-1H-imidazol-1-yl-5-(trifluoromethyl)phenyl]-3-guanidino-benzamide of the formula (V)

To a suspension of 80g (0.213moles) of 4-methyl-N-[3-(4-methyl-1H-imidazol-1-yl-5-(trifluoromethyl)phenyl]-3-amino-benzamide of the formula (IV) obtained from step (b) in 480ml n-butanol 20.2ml of concentrated hydrochloric acid was added. A solution of 18g (0.427moles) of cyanamide in 18ml water was added and reaction mass was heated to 90-95°C for 20 hours while maintaining pH at 2-3 with Conc. HCl (22ml). Reaction mass was cooled to 10-15°C, filtered and washed with chilled n-butanol. Wet solid was charged into 1.5L purified water and, basified with 40% aqueous sodium hydroxide solution. Reaction mass was maintained at room temperature for 2hours, filtered, washed with water and dried at 60-65°C to yield compound of formula (IV)
Yield: 86.3g (97%)
IR and NMR were consistent with the proposed structure.

### (d) Preparation of Nilotinib of the formula (I)

A mixture of 65g (0.156moles) of 4-methyl-N-[3-(4-methyl-1H-imidazol-1-yl-5-(trifluoromethyl)phenyl]-3-guanidino-benzamide from step (c), 30.0g(0.171moles) of 3-dimethylamino-1-(3-pyridyl)-2-propen-1-one of the formula (VIII) in 650 ml n-butanol was heated at 110-115°C for 9 hours. Reaction mass was brought to room temperature and the separated solid (70g) was filtered off. Wet solid was leached with hot water (700ml) and hot methanol (700ml) successively. The wet product was dried at 60-65°C under vacuum to yield compound of formula (I).
Yield: 52.1g (63%).
MR: 235-236°C
Purity: 99.0% (by HPLC)
IR and NMR were consistent with the proposed structure.

### (e) Preparation of Nilotinib hydrochloride

Into 1L four neck flask a mixture of methanol (500ml) and purified water (52.5ml) was charged. Nilotinib (30g) obtained from step(d) was charged into the flask. Concentrated hydrochloric acid (5.51g) was dissolved in methanol (30ml) and added to reaction mass for 15 minutes. Reaction mass was heated to reflux temperature and maintained at the same temperature for 15 minutes. Reaction mass was brought to 55-60°C and clarified with activated carbon using mixture of methanol (30ml) and purified water (3.25ml) for washing. The clear solution was cooled to 0-5°C and maintained at the same temperature for 3-4 hours. Thus formed Nilotinib hydrochloride was filtered, washed with chilled Isopropanol (30ml) and dried in vacuum tray drier at 40-45°C.

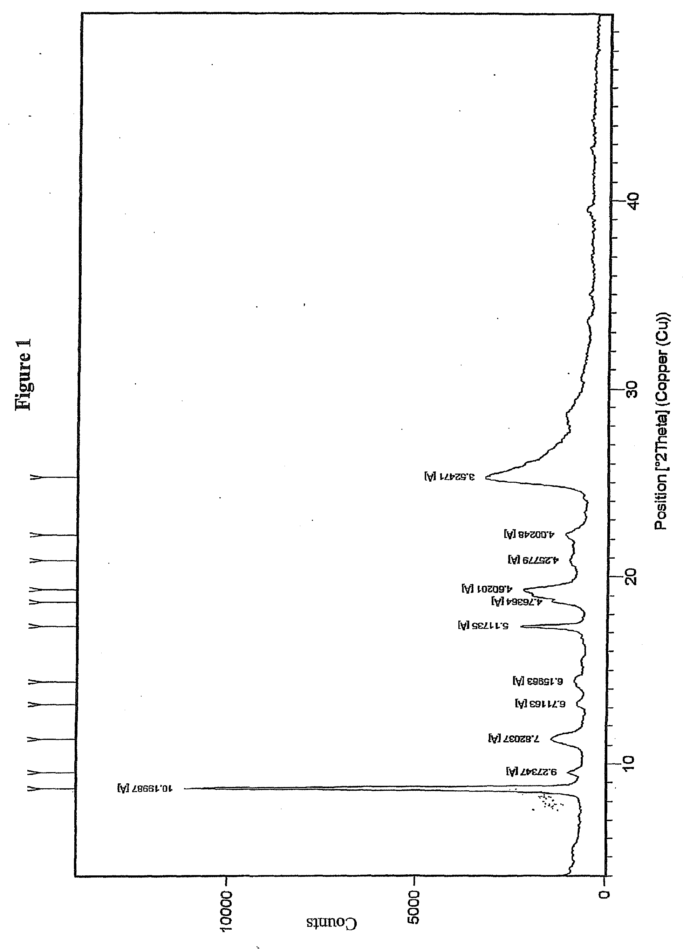

This compound exhibits XRD pattern as depicted by Figure 1. These data are set out in Table 1 below:
Yield: 26.3g
Hydrochloride content: 6.26%
Moisture content by karl-fischer : 5.29%wt/wt

**Table 1**

| **Peak position (degrees 2θ)** | **D-spacing (Å)** | **Relative intensity (%)** |
|---|---|---|
| 8.6623 | 10.19987 | 100.00 |
| 9.5295 | 9.27347 | 2.82 |
| 11.3055 | 7.82037 | 7.67 |
| 13.1808 | 6.71163 | 1.59 |
| 14.3680 | 6.15963 | 1.99 |
| 17.3149 | 5.11735 | 15.92 |
| 18.6166 | 4.76364 | 6.40 |
| 19.2714 | 4.60201 | 13.61 |
| 20.8462 | 4.25779 | 1.41 |
| 22.1922 | 4.00248 | 3.30 |
| 25.2469 | 3.52471 | 22.25 |

### Example 2

Pure nilotinib hydrochloride (1 gm) prepared by the process described in Example 1(e) was taken in a boiling test tube and heated gradually in oil bath maintained at 60-70°C . The substance was examined by XRPD. The results are set out in Table 2.

**Table 2**

| **Polymorph content before heating** | **Time of heating (hours)** | **Polymorph form detected after heating** |
|---|---|---|
| Nilotinib hydrochloride having XRD characterization as in Figure 1 | 10 | Nilotinib hydrochloride having XRD characterization as in Figure 1. |
| | | XRD pattern unchanged |
| Nilotinib hydrochloride having XRD characterization as in Figure 1. | 20 | Nilotinib hydrochloride having XRD characterization as in Figure 1. |
| | | XRD pattern unchanged |
| Nilotinib hydrochloride having XRD characterization as in Figure 1. | 30 | Nilotinib hydrochloride having XRD characterization as in Figure 1. |
| | | XRD pattern unchanged |
| Nilotinib hydrochloride having XRD characterization as in Figure 1. | 40 | Nilotinib hydrochloride having XRD characterization as in Figure 1. |
| | | XRD pattern unchanged |

The above data suggest that Nilotinib hydrochloride prepared by Example 1(e) is not meta stable and is stable to heat even at 70°C/40 hours

### Example 3

The following Table 3 illustrates the formulation composition of Nilotinib hydrochloride prepared from step-(e) of Example 1

**Table 3**

| Components | mg/capsule |
|---|---|
| Nilotinib hydrochloride | 241.00 |
| MCC pH 102 | 107.00 |
| Calsium CMC | 30.00 |
| PG Starch | 15.00 |
| Sodium stearyl fumarate | 7.00 |

### Procedure

Required quantities of Nilotinib hydrochloride and MCC pH 102 were accurately weighed and sifted through #40 meshes and mixed well. Calcium CMC and PG starch were sifted through #40 mesh and mixed to the above blend. This blend was lubricated with #40 mesh sodium stearyl fumarate and submitted for encapsulation.

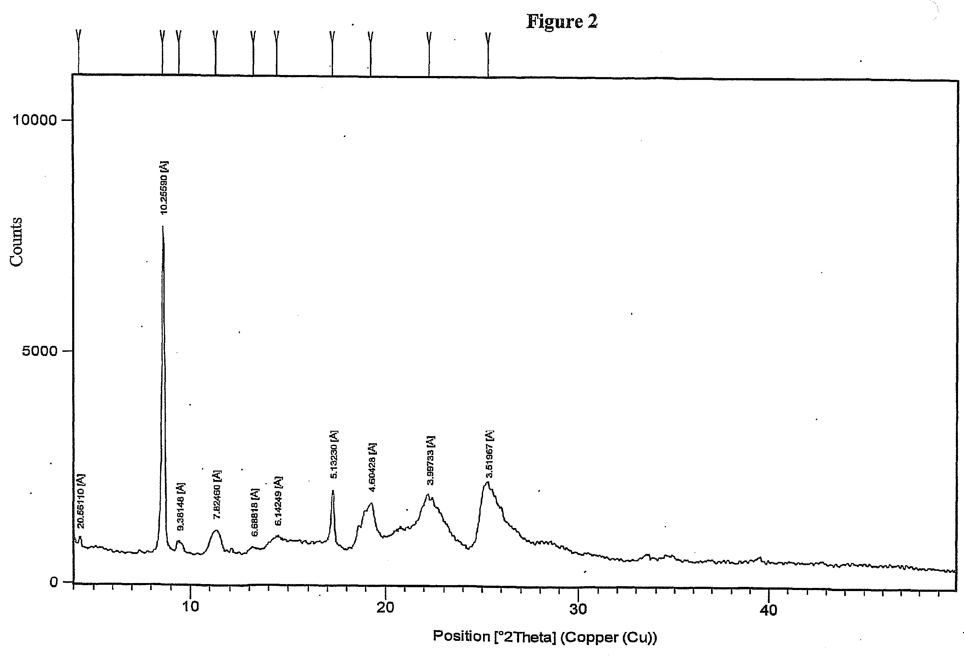

This nilotinib hydrochloride formulation exhibits XRD pattern as depicted by Figure 2. These data are set out in Table 4.

**Table 4**

| **Peak position (degrees 2θ**) | **D-spacing (Å)** | **Relative intensity (%)** |
|---|---|---|
| 4.2941 | 20.56110 | 1.51 |
| 8.6148 | 10.25590 | 100.00 |
| 9.4195 | 9.38148 | 3.76 |
| 11.2994 | 7.82460 | 7.10 |
| 13.2272 | 6.68818 | 1.47 |
| 14.4083 | 6.14249 | 4.59 |
| 17.2641 | 5.13230 | 18.47 |
| 19.2618 | 4.60428 | 14.32 |
| 22.2212 | 3.99733 | 15.90 |
| 25.2836 | 3.51967 | 18.34 |

### Example 4 - stability under high humidity conditions

For illustration of non-hygroscopic nature of Nilotinib hydrochloride drug substance from Example 1 and capsules from Example 3 were kept in stability chambers maintained at 40°C / 75% RH and the water contents of the samples were determined by karl-fischer method. The results are set out in Table 5 below.

**Table 5**

| **Sample** | **Initial moisture Content (%)** | **Final moisture content after 60 days 40°C at humidity of 75%** |
|---|---|---|
| Nilotinib hydrochloride drug stance prepared from example-1 | 5.26 | 5.2% |
| Nilotinib hydrochloride capsules prepared from example-3 | 6.4 | 6.2% |

The above table shows that Nilotinib hydrochloride from Example 1 and nilotinib hydrochlode capsules from Example 3 are non-hygroscopic and have substantial stability even under humidity conditions.

### Example 5

The following Table 6 shows the stability of Nilotinib hydrochloride capsule formulation and drug substances under accelerated stress conditions (40±2°C, 75 ±5% RH, 6 months).

**Table 6**

| **Polymorph characterizing of Nilotinib hydrochloride formulation (Example 3)** | **Polymorph of characterizing Nilotinib hydrochloride API (Example 1)** | **Duration of storage (months) at 40±2°C / 75 ±5% RH** |
|---|---|---|
| **Polymorph form detected by XRD** | **Polymorph form detected by XRD** | |
| Figure 2 | Figure 1 | 0 Month |
| Figure 2 | Figure 1 | 1 Month |
| Figure 2 | Figure 1 | 2 Months |
| Figure 2 | Figure 1 | 3 Months |

The XRD spectra remained essentially the same and unchanged. The stability of Nilotinib hydrochloride API and formulation in accelerated high humidity stress conditions is thus established.

## Claims

1. A process for the preparation of a hydrochloride dihydrate salt of a compound of formula (I): 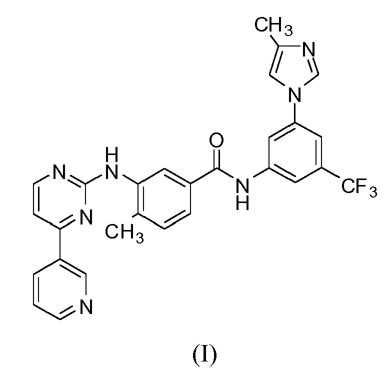 which process comprises converting a compound of formula (IV): 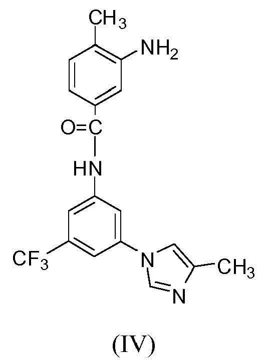 or a pharmaceutically acceptable salt thereof, into the hydrochloride dihydrate salt of the compound of formula (I), wherein hydrochloric acid in a methanol and water medium is used to form the hydrochloride dihydrate salt.

2. A process according to claim 1, which comprises:
(a) reacting the compound of formula (IV), or a pharmaceutically acceptable salt thereof, with cyanamide to obtain a compound of formula (V): 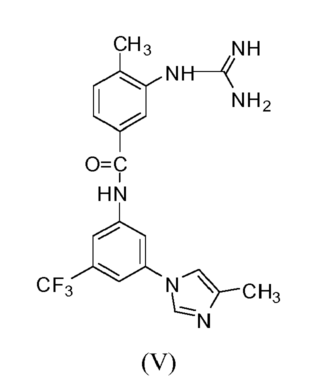 or a pharmaceutically acceptable salt thereof;
(b) reacting the compound of formula (V), or a pharmaceutically acceptable salt thereof, with a compound of formula (VIII): 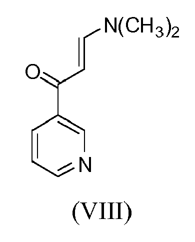 or a pharmaceutically acceptable salt thereof, to obtain the compound of formula (I); and
(c) salifying the compound of formula (I) thus obtained with hydrochloric acid in a methanol and water medium, to form the hydrochloride dihydrate salt.

3. A process according to claim 2, which process comprises preparing the compound of formula (IV) or a pharmaceutically acceptable salt thereof by reducing the nitro moiety of a compound of formula (III) 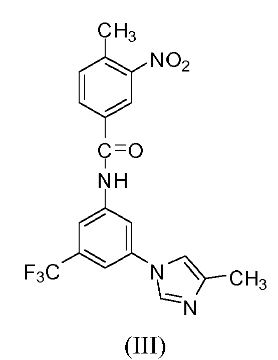 or a pharmaceutically acceptable salt thereof.

4. A process according to claim 3, which process comprises preparing the compound of formula (III), or a pharmaceutically acceptable salt thereof, by reacting a compound of formula (XI): 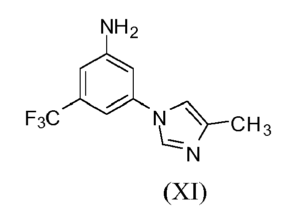 or a pharmaceutically acceptable salt thereof, with a compound of formula (IIA): 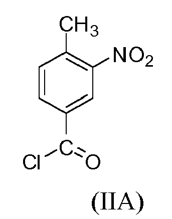 or a pharmaceutically acceptable salt thereof.

5. A process according to any one of the preceding claims, wherein the hydrochloride dihydrate salt has a moisture content of from 5% by weight to 7% by weight.

## Patentansprüche

1. Verfahren zur Herstellung eines Hydrochlorid-Dihydrat-Salzes einer Verbindung der Formel (I): 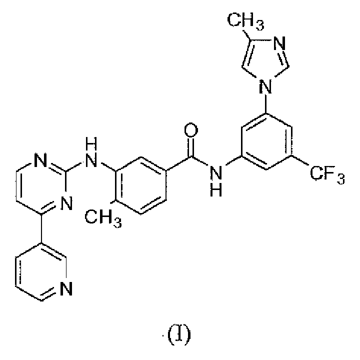 wobei das Verfahren umfasst: das Umwandeln einer Verbindung der Formel (IV): 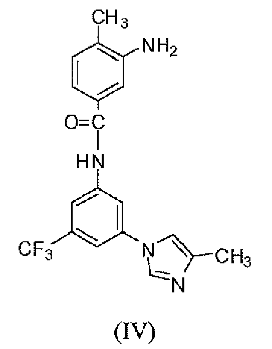 oder eines pharmazeutisch annehmbaren Salzes davon in das Hydrochlorid-Dihydrat-Salz der Verbindung der Formel (I), wobei Salzsäure in einem Methanol-und-Wasser-Medium verwendet wird, um das Hydrochlorid-Dihydrat-Salz zu bilden.

2. Verfahren nach Anspruch 1, welches umfasst:
(a) das Umsetzen einer Verbindung der Formel (IV) oder eines pharmazeutisch annehmbaren Salzes davon mit Cyanamid, um eine Verbindung der Formel (V): 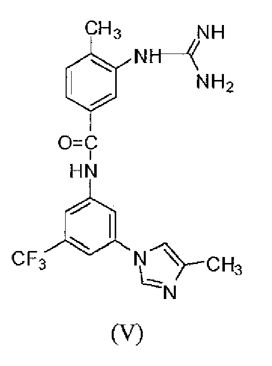 oder ein pharmazeutisch annehmbares Salz davon zu erhalten;
(b) das Umsetzen der Verbindung der Formel (V) oder eines pharmazeutisch annehmbaren Salzes davon mit einer Verbindung der Formel (VIII): 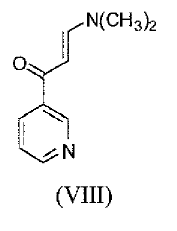 oder einem pharmazeutisch annehmbaren Salz davon, um die Verbindung der Formel (I) zu erhalten; und
(c) Das Salifizieren der so erhaltenen Verbindung der Formel (I) mit Salzsäure in einem Methanol-und-Wasser-Medium, um das Hydrochlorid-Dihydrat-Salz zu bilden.

3. Verfahren nach Anspruch 2, wobei das Verfahren umfasst:
das Herstellen der Verbindung der Formel (IV) oder eines pharmazeutisch annehmbaren Salzes davon durch Reduzieren der Nitrogruppe einer Verbindung der Formel (III) 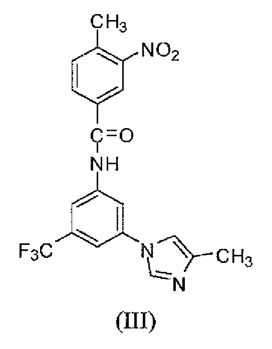
oder eines pharmazeutisch annehmbaren Salzes davon.

4. Verfahren nach Anspruch 3, wobei das Verfahren umfasst:
das Herstellen der Verbindung der Formel (III) oder eines pharmazeutisch annehmbaren Salzes davon durch Umsetzen einer Verbindung der Formel (XI): 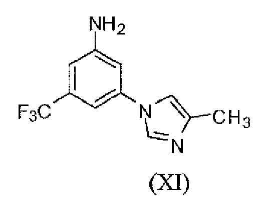 oder eines pharmazeutisch annehmbaren Salzes davon mit einer Verbindung der Formel (IIA): 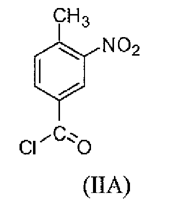 oder einem pharmazeutisch annehmbaren Salz davon.

5. Verfahren nach einem der voranstehenden Ansprüche, wobei das Hydrochlorid-Dihydrat-Salz einen Feuchtigkeitsgehalt von 5 Gew.-% bis 7 Gew.-% aufweist.

## Revendications

1. Procédé de préparation du sel chlorhydrate dihydraté du composé de formule (I) : 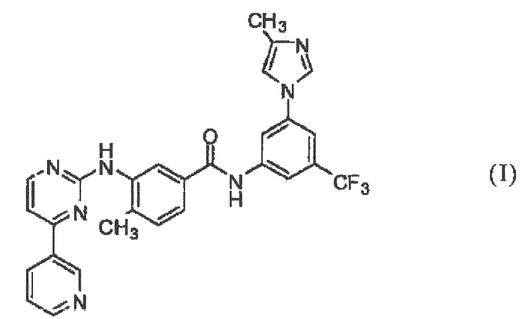 lequel procédé comporte la conversion du composé de formule (IV) : 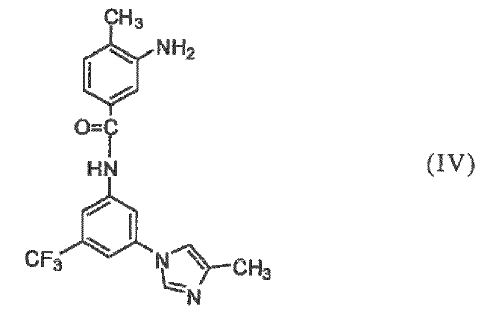 ou d'un sel pharmacologiquement admissible de celui-ci, en le dihydrate du sel chlorhydrate du composé de formule (I), et dans lequel procédé on utilise de l'acide chlorhydrique dans un milieu de méthanol et d'eau pour former le dihydrate de sel chlorhydrate.

2. Procédé conforme à la revendication 1, qui comporte les étapes suivantes :
a) faire réagir le composé de formule (IV), ou un sel pharmacologiquement admissible de celui-ci, avec du cyanamide, de manière à obtenir le composé de formule (V) : 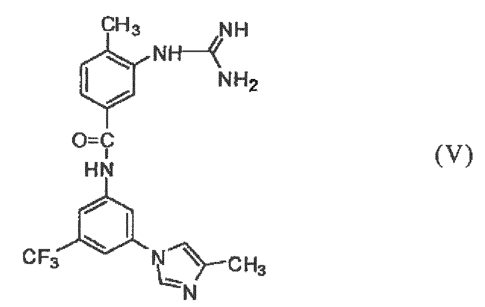 ou un sel pharmacologiquement admissible de ce composé ;
b) faire réagir le composé de formule (V), ou son sel pharmacologiquement admissible, avec le composé de formule (VIII) : 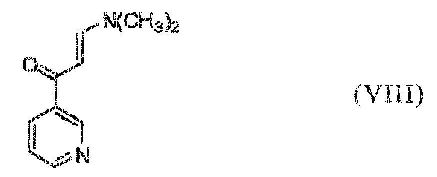 ou avec un sel pharmacologiquement admissible de celui-ci, de manière à obtenir le composé de formule (I) ;
c) et salifier le composé de formule (I) ainsi obtenu, avec de l'acide chlorhydrique dans un milieu de méthanol et d'eau, de manière à obtenir le dihydrate du sel chlorhydrate.

3. Procédé conforme à la revendication 2, lequel procédé comporte le fait de préparer le composé de formule (IV), ou un sel pharmacologiquement admissible de celui-ci, en réduisant le groupe nitro du composé de formule (III) : 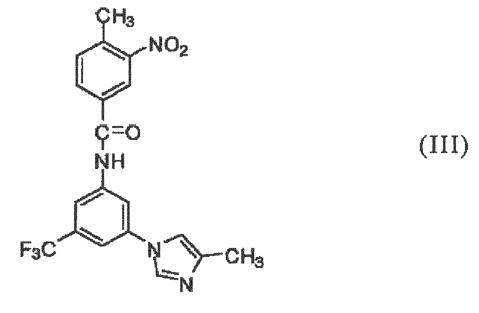 ou d'un sel pharmacologiquement admissible de ce composé.

4. Procédé conforme à la revendication 3, lequel procédé comporte le fait de préparer le composé de formule (III), ou un sel pharmacologiquement admissible de celui-ci, en faisant réagir le composé de formule (XI) : 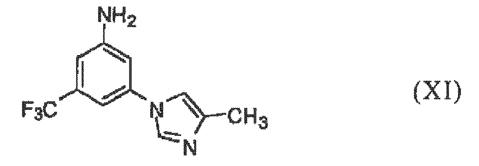 ou un sel pharmacologiquement admissible de ce composé, avec le composé de formule (IIA) 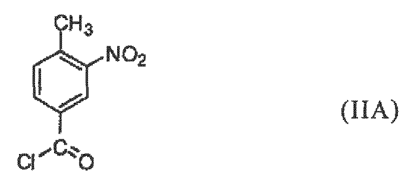 ou avec un sel pharmacologiquement admissible de ce composé.

5. Procédé conforme à l'une des revendications précédentes, dans lequel le dihydrate de sel chlorhydrate présente une teneur en humidité qui vaut de 5 % en poids à 7 % en poids.
